(19)

Europäisches Patentamt

European Patent Office

Office européen des brevets

(11) **EP 4 379 027 A2**

(12) **EUROPEAN PATENT APPLICATION**

(43) Date of publication:
**05.06.2024 Bulletin 2024/23**

(21) Application number: **23209072.0**

(22) Date of filing: **10.11.2023**

(51) International Patent Classification (IPC):
***C10G 11/05*** (2006.01)      ***C10G 11/18*** (2006.01)
***C10G 1/10*** (2006.01)

(52) Cooperative Patent Classification (CPC):
**C10G 11/18; C10G 11/05;** C10G 1/10;
C10G 2400/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.11.2022 KR 20220150450**

(71) Applicants:
• **SK Innovation Co., Ltd.
Seoul 03188 (KR)**
• **SK Geo Centric Co., Ltd.
Jongno-gu
Seoul 03161 (KR)**

(72) Inventors:
• **JEON, Hee Jung
34124 Daejeon (KR)**
• **KIM, Ok Youn
34124 Daejeon (KR)**
• **LEE, Ho Won
34124 Daejeon (KR)**

(74) Representative: **Frick, Robert
Lorenz Seidler Gossel
Rechtsanwälte Patentanwälte
Partnerschaft mbB
Widenmayerstraße 23
80538 München (DE)**

(54) **METHOD AND DEVICE FOR CONVERTING WASTE PLASTIC PYROLYSIS OIL INTO LIGHT OLEFINS WITH HIGH YIELD**

(57)     The present invention relates to a method for converting waste plastic pyrolysis oil into light olefins with a high yield, the method including: (1) inputting waste plastic pyrolysis oil into a reactor; (2) allowing the waste plastic pyrolysis oil to react in the reactor in the presence of a catalytic cracking catalyst containing a first metal and a second metal; and (3) recovering light olefins by separating the catalytic cracking catalyst and oil from a product obtained in the step (2).

EP 4 379 027 A2

**Description**

TECHNICAL FIELD

The present invention relates to a method and device for converting waste plastic pyrolysis oil into light olefins with a high yield, and more particularly, to a method and device for converting waste plastic pyrolysis oil into light olefins with a high yield that may implement high olefin selectivity and may minimize production of coke.

BACKGROUND

Waste plastics, which are produced using petroleum as a feedstock, are difficult to be recycled and are mostly disposed of as garbage. These wastes take a long time to degrade in nature, which causes contamination of the soil and serious environmental pollution. As a method for recycling waste plastics, there is a method for pyrolyzing and converting waste plastics into usable oil, and the obtained oil is called waste plastic pyrolysis oil.

Meanwhile, in a mixture of hydrocarbon oils such as petroleum-based oil, olefins, in particular, light olefins such as ethylene and propylene have been widely used in the petrochemical industry. Until now, most ethylene or propylene has been mainly produced by pyrolysis of hydrocarbon oils such as natural gas, naphtha oil, and gas oil in a steam atmosphere at a high temperature of 800°C or higher under a catalyst-free condition, but the method described above has problems of low olefin selectivity and low production yield.

As a measure to improve production yield and reaction efficiency such as olefin selectivity or conversion rate, a fluid catalytic cracking (FCC) process has been performed. Representative examples of the FCC process include a catalytic cracking process using an acid catalyst. In particular, among various acid catalysts, zeolites have been most widely used, and as representative zeolites for catalytic cracking, ZSM-5 zeolite, USY zeolite, β-zeolite, and the like have been used. The catalytic cracking process has been performed by inducing a cracking reaction of hydrocarbon oils such as a petroleum-based feedstock by carbenium ions using a zeolite catalyst having a solid acid active site, but since the waste plastic pyrolysis oil is a mixture of hydrocarbon oils mainly having a linear hydrocarbon structure, the waste plastic pyrolysis oil has a relatively lower concentration of carbenium ions than the petroleum-based feedstock, which makes it difficult to induce the cracking reaction. That is, the catalytic cracking process using a zeolite catalyst used in the existing petroleum-based feedstock technology has limitations in being applied to waste plastic pyrolysis oil.

In order to solve this problem, the catalytic cracking process has been performed by introducing an active metal according to the related art, such as nickel, iron, vanadium, palladium, or platinum, which has a strong hydrogen transfer activity capable of dehydrogenation/hydrogenation reaction, into a catalytic cracking catalyst. However, an increase in hydrogen transfer reaction according to the introduction of the active metal may improve the cracking activity, but causes saturation of olefins. Therefore, the olefin selectivity is lowered, such that there is a serious problem in that the production yield of light olefins is significantly reduced.

In addition, a process of converting pyrolysis oil into light olefins is difficult to economically and commercially put into practical use due to a low conversion yield and deterioration of quality caused by deactivation of the catalyst due to coke produced during the pyrolysis process.

Therefore, there is a demand for a technology capable of producing light olefins from waste plastic pyrolysis oil with a high yield.

SUMMARY

The present invention aims to providing a method and device for converting waste plastic pyrolysis oil into light olefins with a high yield that may implement high olefin selectivity and may minimize production of coke.

Against this background, the present invention relates to a method for converting waste plastic pyrolysis oil into light olefins includes: (1) inputting waste plastic pyrolysis oil into a reactor; (2) allowing the waste plastic pyrolysis oil to react in the reactor in the presence of a catalytic cracking catalyst containing a first metal and a second metal; and (3) recovering light olefins by separating the catalytic cracking catalyst and oil from a product obtained in the step (2).

In an exemplary embodiment, the first metal may include at least one metal selected from manganese, tin, and zinc.

In an exemplary embodiment, the second metal may include at least one metal selected from titanium, zirconium, hafnium, niobium, and vanadium.

In an exemplary embodiment, the catalytic cracking catalyst may further contain a zeolite, clay, and a binder.

In an exemplary embodiment, the zeolite may include ZSM-5, ZSM-11, Y-zeolite, ferrierite, mordenite, MCM-22, SUZ-4, or L-type zeolite.

In an exemplary embodiment, a weight ratio of the first metal to the second metal in the catalytic cracking catalyst may be 6:0.1 to 6:1, preferably 6:0.1 to 6:0.8, and more preferably 6:0.2 to 6:0.6.

In an exemplary embodiment, the catalytic cracking catalyst may contain 20 to 70 wt%, preferably 30 to 50 wt% of the zeolite, 10 to 60 wt%, preferably 20 to 40 wt% of the clay, 10 to 50 wt%, preferably 15 to 35 wt% of the binder, 1 to 6 wt%, preferably 1 to 4 wt% of the first metal, and 0.1 to 1 wt%, preferably 0.1 to 0.6 wt% of the second metal. The wt% of the zeolite, clay, binder, first and second metals may add up to 100 wt%.

In an exemplary embodiment, the catalytic cracking catalyst may have an average particle size of 50 to 2,000 pm, preferably 50 to 700 pm, more preferably 80 to 150 $\mu$m. It is measured using a test method based on ASTM standard test method D4464-201.

In an exemplary embodiment, the catalytic cracking catalyst may have a total specific surface area of 50 to 150 m$^2$/g, preferably 70 to 100 m$^2$/g, and/or an apparent density of 0.5 to 1 g/cm$^3$, preferably 0.5 to 0.8 g/cm$^3$.

In an exemplary embodiment, the catalytic cracking catalyst may satisfy the following Expression 1.

$$[\text{Expression 1}]$$

$$2 < (D90-D10)/D50 < 5$$

wherein D10 is a 10% cumulative diameter, D50 is a 50% cumulative diameter, and D90 is a 90% cumulative diameter, and wherein each of D10, D50 and D90 are as determined with laser diffraction according to DIN ISO 13320.

In an exemplary embodiment, the waste plastic pyrolysis oil may contain a vacuum gas oil (VGO) component having a boiling point of 340°C or higher at atmospheric pressure.

In an exemplary embodiment, the reactor may be a fluidized bed reactor.

In an exemplary embodiment, the step (2) is performed at a temperature of 400 to 600°C and a reaction pressure of 50 to 200 kPa.

In another general aspect, a device for converting waste plastic pyrolysis oil into light olefins includes: a fluidized bed reactor configured to receive waste plastic pyrolysis oil and to perform a catalytic cracking reaction; a cyclone configured to receive a product from the fluidized bed reactor and to separate the product into a catalyst and oil; and a stabilizer configured to receive the oil from the cyclone and to separate the oil into a gas component and a liquid component.

The device is preferably configured for carrying out a method of the invention.

Other features and aspects will be apparent from the following detailed description, the drawings, and the claims.

## DETAILED DESCRIPTION OF EMBODIMENTS

Unless otherwise defined, a unit of "%" used in the present specification refers to "wt%".

Unless otherwise defined, a "boiling point" used in the present specification is based on atmospheric pressure, and the expression "bP" or the like refers to a boiling point.

Unless otherwise defined, a unit of "ppm" used in the present specification refers to "mass ppm".

The catalytic cracking process has been performed in the related art by inducing a cracking reaction of hydrocarbon oils such as a petroleum-based feedstock by carbenium ions using a zeolite catalyst having a solid acid active site, but since the waste plastic pyrolysis oil is a mixture of hydrocarbon oils mainly having a linear hydrocarbon structure, the waste plastic pyrolysis oil has a relatively lower concentration of carbenium ions than the petroleum-based feedstock, which makes it difficult to induce the cracking reaction. That is, the catalytic cracking process using a zeolite catalyst used in the existing petroleum-based feedstock technology has limitations in being applied to waste plastic pyrolysis oil.

In order to solve this problem, the catalytic cracking process has been performed by introducing an active metal according to the related art, such as nickel, iron, vanadium, palladium, or platinum, which has a strong hydrogen transfer activity capable of dehydrogenation/hydrogenation reaction, into a catalytic cracking catalyst. However, an increase in hydrogen transfer reaction according to the introduction of the active metal may improve the cracking activity, but causes saturation of olefins. Therefore, the olefin selectivity is lowered, such that there is a serious problem in that the production yield of light olefins is significantly reduced.

In addition, a process of converting pyrolysis oil into light olefins is difficult to economically and commercially put into practical use due to a low conversion yield and deterioration of quality caused by deactivation of the catalyst due to coke produced during the pyrolysis process.

The method of the present invention may implement high olefin selectivity and may minimize production of coke, such that a light olefin conversion yield may be significantly improved. Specifically, the present invention provides a method for converting waste plastic pyrolysis oil into light olefins, the method including: (1) inputting waste plastic pyrolysis oil into a reactor; (2) allowing the waste plastic pyrolysis oil to react in the reactor in the presence of a catalytic cracking catalyst containing a first metal and a second metal; and (3) recovering light olefins by separating the catalytic cracking catalyst and oil from a product obtained in the step (2).

The waste plastic pyrolysis oil includes a mixture of hydrocarbon oils, and includes, for example, a mixture of hydrocarbon oils having various boiling points and molecular weight distributions such as C7 to C9 Naphtha having a boiling point of 80 to 150°C at atmospheric pressure, C10 to C17 Kero having a boiling point of 150 to 265°C, C18 to C20 LGO having a boiling point of 265 to 340°C, and C21 or more VGO/AR having a boiling point of 340°C or higher. As described below, in the related art, VGO has conversion efficiency that is much lower than those of Kero and Naphtha in a light olefin conversion process, making it difficult to use VGO, and therefore, the conversion process has been performed mainly using Kero and Naphtha as a feedstock excluding VGO. On the other hand, a light olefin high-yield conversion process of the present invention is excellent in cracking efficiency and light olefin conversion efficiency even when waste plastic pyrolysis oil containing a vacuum gas oil (VGO) component is used as a feedstock.

In an exemplary embodiment, the first metal may include at least one metal selected from manganese, tin, zinc, and cobalt.

In an exemplary embodiment, the second metal may include at least one metal selected from titanium, zirconium, hafnium, niobium, and vanadium.

As the catalytic cracking catalyst containing both the first metal and the second metal controls the hydrogen transfer activity (or hydrogen transfer reaction activity) at optimum efficiency, a coke production reaction of the produced olefins may be suppressed, and the olefins may be prevented from being saturated, such that a paraffin production reaction may be suppressed. Therefore, the olefin selectivity is high, and the amount of coke produced is reduced, such that the catalytic deactivation may be prevented, and as a result, light olefins may be obtained from waste plastic pyrolysis oil with a high yield. Preferably, in terms of the effect, the first metal may be manganese or tin, and the second metal may be zirconium or titanium. More preferably, the first metal may be manganese, and the second metal may be zirconium. In this case, the olefin selectivity and the coke reduction effect may be most excellent.

In an exemplary embodiment, the catalytic cracking catalyst may further contain a zeolite, clay, and a binder. As the catalytic cracking catalyst further contains a zeolite, clay, and a binder, a composite is formed, and thus the catalytic cracking catalyst may have high mechanical strength and may be used in a large-scale petrochemical process such as a process of upgrading waste plastic pyrolysis oil.

In an exemplary embodiment, the zeolite may include ZSM-5, ZSM-11, Y-zeolite, ferrierite, mordenite, MCM-22, SUZ-4, or L-type zeolite. A zeolite is a porous molecular sieve having a rich pore structure and a large specific surface area, and has a lot of active sites and excellent catalytic cracking efficiency. The zeolite may control the cracking activity according to the number of atoms constituting the pores, a pore size, or a stereostructure (one-dimensional, two-dimensional, or three-dimensional structure) of the pores. Preferred zeolites include ZSM-5 or Y-zeolite.

The catalytic cracking catalyst may contain a binder as a binding agent. The binder may contain $Al_2O_3$, $SiO_2$, or $Al_2O_3$-$SiO_2$, but this is merely an example, and the binder is not limited thereto.

The clay may comprise kaolin or a mixture of kaolin and montmorillonite. In this case, Clay has a characteristic of having a weak acid site and can crack the heavy hydrocarbon at high temperatures. The weak acid site means an acid site at which ammonia can be desorbed at 400°C or less at ammonia TPD analysis. Also, the clay used is good when the surface area is large and the mesopores are well developed, but the clay of low surface area does not mean that it cannot be used.

In an exemplary embodiment, a weight ratio of the first metal to the second metal in the catalytic cracking catalyst may be 6:0.1 to 6:1. When the above weight ratio is satisfied, the olefin selectivity and the coke reduction effect may be optimized, and as a result, olefins may be obtained with a high yield. When the weight ratio of the first metal to the second metal is 6:0.1 to 6:1 or more, the effect of improving olefin selectivity may be rather reduced due to an increase in content of the second metal. Specifically, the weight ratio may be 6:0.1 to 6:0.8, and more specifically, may be 6:0.2 to 6:0.6.

In an exemplary embodiment, the catalytic cracking catalyst may contain 20 to 70 wt% of the zeolite, 10 to 60 wt% of the clay, 10 to 50 wt% of the binder, 1 to 6 wt% of the first metal, and 0.1 to 1 wt% of the second metal. When the catalytic cracking catalyst satisfies all the above weight ranges, the light olefin conversion yield may be significantly improved, and the catalytic deactivation may be effectively prevented, and as a result, the fluid catalytic cracking process may be stably performed for a long time.

Each component contained in the catalytic cracking catalyst will be individually described. When each component is contained within the above range of wt% on the premise of satisfying the weight ratio of the first metal to the second metal of 6:0.1 to 6:1, the cracking activity and the olefin selectivity are controlled due to a weak hydrogen transfer reaction, such that the light olefin conversion yield may be maximized. Specifically, the first metal and the second metal may be contained in amounts of 1 to 5 wt% and 0.1 to 0.8 wt%, respectively, and more specifically, the first metal and the second metal may be contained in amounts of 1 to 4 wt% and 0.1 to 0.6 wt%, respectively.

When the zeolite is contained in the weight range, the catalytic cracking efficiency may be improved, and the light olefin conversion yield may be improved. Specifically, the zeolite may be contained in an amount of 20 to 60 wt%, and more specifically, may be contained in an amount of 30 to 50 wt%.

When the clay is contained in the above weight range, the weight of the clay with respect to the catalyst and the overall catalytic activity may be optimized. Specifically, the clay may be contained in an amount of 10 to 50 wt%, and more specifically, may be contained in an amount of 20 to 40 wt%.

When the binder is contained in the above weight range, physical properties such as abrasion strength of the catalytic cracking catalyst may be excellently maintained. Specifically, the binder may be contained in an amount of 10 to 40 wt%, and more specifically, may be contained in an amount of 15 to 35 wt%.

In an exemplary embodiment, the catalytic cracking catalyst may have an average particle size of 50 to 2,000 pm. In a case where a catalytic cracking catalyst having the above size is screened and used to correspond to intrinsic properties of waste plastic pyrolysis oil, such as a viscosity and a density, the olefin selectivity and the catalytic activity may be significantly improved. Specifically, the average particle size may be 50 to 1,000 pm, and more specifically, may be 50 to 700 pm. Preferably, the average particle size may be 50 to 200 pm, and more preferably, may be 80 to 150 pm.

The catalytic cracking catalyst may have a total specific surface area of 50 to 150 $m^2$/g and an apparent density of 0.5 to 1 g/$cm^3$. Within the above ranges, a contact area with the feedstock is optimized, such that the catalytic cracking efficiency may be improved. Specifically, the total specific surface area may be 50 to 130 $m^2$/g and the apparent density may be 0.5 to 0.8 g/$cm^3$, and more specifically, the total specific surface area may be 70 to 100 $m^2$/g and the apparent density may be 0.6 to 0.7 g/$cm^3$.

In an exemplary embodiment, the catalytic cracking catalyst may satisfy the following Expression 1.

$$[\text{Expression 1}]$$

$$2 < (D90-D10)/D50 < 5$$

Expression 1 represents a particle size distribution width of the catalytic cracking catalyst in a volume-based distribution measured with a laser diffraction particle size distribution measuring device, according to DIN ISO 13320, D10 is a 10% cumulative diameter, D50 is a 50% cumulative diameter, and D90 is a 90% cumulative diameter.

When Expression 1 is not satisfied, problems such as a decrease in stability and a decrease in catalytic cracking efficiency of the catalytic cracking catalyst may occur due to a decrease in slurry dispersibility. As the catalytic cracking catalyst satisfies Expression 1, the stability and the catalytic cracking efficiency of the catalyst may be improved. Specifically, Expression 1 may be 2 < (D90-D10)/D50 < 4, and more specifically, Expression 1 may be 2 < (D90-D10)/D50 < 3. As the particle size distribution width satisfies the above numerical range, the stability and the catalytic cracking efficiency may be significantly improved more preferably in a fluidized bed reactor.

In an exemplary embodiment, the waste plastic pyrolysis oil may contain a vacuum gas oil (VGO) component having a boiling point of 340°C or higher at atmospheric pressure. In the related art, VGO has conversion efficiency that is much lower than those of Kero and Naphtha in a light olefin conversion process, making it difficult to use VGO, and therefore, the conversion process has been performed mainly using Kero and Naphtha as a feedstock excluding VGO. On the other hand, the light olefin high-yield conversion process of the present invention is excellent in cracking efficiency and light olefin conversion efficiency even when waste plastic pyrolysis oil containing a vacuum gas oil (VGO) component is used as a feedstock.

In an exemplary embodiment, the reactor may be a fluidized bed reactor. In a case where a fixed bed reactor is used, although a yield of olefins is high at the beginning of the reaction in which hydrocarbons are in contact with the catalytic cracking catalyst, deactivation of the catalytic cracking catalyst and excessive production of coke occur over time, such that a conversion rate of hydrocarbons and the yield of olefins are reduced as a whole, and a lot of energy is consumed in a regeneration process. By using a fluidized bed reactor, it is possible to solve the above problems and economically and efficiently produce light olefins.

In an exemplary embodiment, the step (2) is performed at a temperature of 400 to 600°C and a reaction pressure of 50 to 200 kPa. Since the reaction efficiency in the step (2) is highly dependent on the temperature and pressure, the energy consumption may be minimized under the above conditions, and deactivation of the catalytic cracking catalyst may be effectively suppressed. Specifically, the temperature may be 400 to 550°C and the pressure may be 50 to 150 kPa, and more specifically, the temperature may be 400 to 500°C and the pressure may be 50 to 100 kPa.

In addition, the reaction efficiency in the step (2) may be dependent on a plateau time, a catalyst/pyrolysis oil ratio, or a pyrolysis oil/steam ratio. The plateau time may be 0.1 to 600 seconds, the catalyst/pyrolysis oil ratio may be 1 to 50, and the pyrolysis oil/steam ratio may be 0.01 to 10, specifically, the plateau time may be 0.5 to 120 seconds, the catalyst/pyrolysis oil ratio may be 5 to 30, and the pyrolysis oil/steam ratio may be 0.1 to 2.0, and more specifically, the plateau time may be 1 to 20 seconds, the catalyst/pyrolysis oil ratio may be 10 to 20, and the pyrolysis oil/steam ratio may be 0.3 to 1.

After performing the catalytic cracking reaction, light olefins may be recovered by separating the catalytic cracking catalyst and oil from a reaction product through the step (3). Specifically, a reaction product produced in the step (2) may be introduced into a cyclone described below, and the reaction product and the catalytic cracking catalyst may be separated within a short time. As described above, in the step (3), the catalyst and oil are separated to recover light olefins, such that light olefins may be finally obtained from the waste plastic pyrolysis oil.

A method for preparing a catalytic cracking catalyst may be as follows. The method for preparing a catalytic cracking catalyst may include: preparing a binder containing alumina gel; preparing a solid fine powder by mixing a zeolite and clay; preparing a mixed slurry by uniformly mixing the mixed solid fine powder and the binder; preparing a spherical catalyst by spray-drying the mixed slurry and then performing calcination; recovering a catalyst having an average particle size of 5 to 200 μm through sieving; ion-exchanging the recovered catalyst with an aqueous solution of rear earth metal (RE) chloride containing cerium and lanthanum and then performing calcination; and supporting an aqueous solution of a first metal precursor and a second metal precursor on a surface of the catalyst and then performing calcination.

A yield of converting the waste plastic pyrolysis oil into light olefins may be 5% or more. When the catalytic cracking catalyst containing a first metal and a second metal is used, a yield of converting the waste plastic pyrolysis oil into light olefins is 5% or more, and thus, light olefins may be obtained with a high yield. Specifically, the light olefin conversion yield may be 10% or more.

In addition, the present invention provides a device for converting waste plastic pyrolysis oil into light olefins, the device including: a fluidized bed reactor configured to receive waste plastic pyrolysis oil and to perform a catalytic cracking reaction; a cyclone configured to receive a product from the fluidized bed reactor and to separate the product into a catalyst and oil; and a stabilizer configured to receive the oil from the cyclone and to separate the oil into a gas component and a liquid component.

The waste plastic pyrolysis oil is fed into the fluidized bed reactor, and may be fed after being heated to a temperature of 30 to 600°C for a smoother reaction. The waste plastic pyrolysis oil may be mixed with a catalytic cracking catalyst provided in the fluidized bed reactor, or may be mixed with a regenerated catalyst fed through a pipe connected to the fluidized bed reactor from a regenerator.

In the fluidized bed reactor, the catalytic cracking reaction may be performed at a temperature of 400 to 600°C and a pressure of 50 to 200 kPa, and a reaction product produced therefrom and the catalyst may be introduced into the cyclone and then separated. The cyclone may be optionally used to increase the efficiency of the separation process. The separated reaction product is introduced into the stabilizer from the cyclone, and may be separated into a gas component and a liquid component through cooling.

Hereinafter, the present invention will be described in detail with reference to Examples.

Example 1

1) Feedstock

200 g of waste plastics were fed to a batch pyrolysis reactor, and pyrolysis was performed at 500°C, thereby obtaining pyrolysis oil. The distribution of the mixture of hydrocarbon oils included in the pyrolysis oil is shown in Table 1.

[Table 1]

| Cut Name | Predictive carbon range | Boiling point (°C) | wt% |
|---|---|---|---|
| Naphtha | C7 to C9 | 80 to 150 | 22.1 |
| KERO | C10 to C17 | 150 to 265 | 28.1 |
| LGO | C18 to C20 | 265 to 340 | 15.9 |
| VGO | C21 or more | > 340 | 33.9 |
| Sum | - | - | 100 |

The pyrolysis oil was separated by boiling point through a distiller, and only C21+ VGO was selectively recovered, thereby preparing a pyrolysis oil feedstock.

2) Preparation of Catalyst

70 g of Pseudo-boehmite was introduced into 500 g of water, 10 g of formic acid was introduced while stirring the mixture at room temperature, and then the mixture was maintained for 3 hours, thereby preparing alumina gel. Clay(Kaoline) and ZSM-5 were introduced into a mixer and then stirred for 10 minutes. ZSM-5 was introduced into clay in an amount of 43 wt%. After the mixture of clay, ZSM-5, and MgO was stirred in the mixer to make the mixture into uniform small particles, the alumina gel was introduced, and then stirring was performed again with the mixer. 60 g of Ludox (AS 40) was introduced during stirring, and then a viscosity of the slurry was measured using a viscometer.

Thereafter, in the process of converting the viscosity of the slurry from sol to gel, the slurry was prepared into a spherical catalyst through spray drying, the spherical catalyst was dried in an oven at 120°C for 12 hours, and then the dried spherical catalyst was calcined at 550°C for 3 hours, thereby preparing a fluidized bed catalyst. The recovered catalyst was separated by screening only a catalyst having a particle size of 30 to 200 $\mu$m through sieving, the screened catalyst was subjected to ion-exchange with a 5% RE-metal solution at 60°C for 3 hours, the catalyst was dried, and then the dried catalyst was calcined at 550°C for 3 hours, thereby preparing an ion-exchanged fluidized bed catalyst. $MnCl_2 \cdot 4H_2O$ and $ZrO(NO_3)_2 \cdot H_2O$ dissolved in a predetermined amount of water and 1% $HNO_3$ introduced based on the total aqueous solution were supported on the recovered catalyst by an incipient wetness method so that 3 wt% of manganese (Mn) and 0.1 wt% of zirconium (Zr) were introduced. The supported catalyst was dried at 120°C and then calcined at 500°C for 3 hours.

3) Catalytic Cracking Process

The catalytic activity was evaluated by a davison circulating riser (DCR) reaction system. A cracking reaction and a catalyst regeneration reaction in a regenerator were performed at 530°C and 730°C, respectively, and the reaction was performed while a stripper where a cracking reaction occurred was maintained at 527°C. A feedstock and steam were introduced into a reaction unit at 850 g/h and 80 g/h, respectively, $N_2$ was introduced at a total rate of 220 lps by introducing $N_2$ into a stripper at 180 lps and into a reactor at 40 lps, and the catalyst separated in a cyclone was introduced into a regenerator to be regenerated. Air was introduced at a rate of 900 lps for regeneration of the catalyst in the regenerator. The operation was performed under a condition in which a Cat/Oil ratio, which was a ratio of the catalyst to the feedstock introduced, was set to 11 to 20. A product was separated into a gas and a liquid in a stabilizer, and then the recovered gas was analyzed through GC, and the liquid was analyzed through SIMDIST. Coke, $CO_2$, and $H_2$ were analyzed by a $CO/CO_2$ analyzer.

Example 2

A process was performed under the same conditions as those of Example 1, except that a catalytic cracking catalyst was prepared using Ti $[OCH(CH_3)_2]_4$ instead of $ZrO(NO_3)_2 \cdot H_2O$ in the preparation of the catalytic cracking catalyst.

Example 3

A process was performed under the same conditions as those of Example 1, except that a catalytic cracking catalyst was prepared using $SnCl_2 \cdot 2H_2O$ instead of $MnCl_2 \cdot 4H_2O$ in the preparation of the catalytic cracking catalyst.

Example 4

A process was performed under the same conditions as those of Example 1, except that a catalytic cracking catalyst was prepared so that 3 wt% of Mn and 0.6 wt% of Zr were supported.

**Comparative Example 1**

A process was performed under the same conditions as those of Example 1, except that a catalytic cracking catalyst was prepared without using ZrO(NO3)2·H2O.

**Comparative Example 2**

A process was performed under the same conditions as those of Example 1, except that a catalytic cracking catalyst was prepared without using MnCl2·4H2O.

**Evaluation Examples**

The gas component oil was quantified through on-line gas chromatography (model name: HP 6890N), the liquid component oil was recovered in a storage tank and then quantified through SIMDIST, and then a light olefin conversion yield was evaluated by analyzing weights of light olefins including ethylene and propylene and coke from the waste plastic pyrolysis oil.

The analysis results are shown in Table 2.

[Table 2]

|  | Example 1 | Example 2 | Example 3 | Example 4 | Comparative Example 1 | Comparative Example 2 |
|---|---|---|---|---|---|---|
| First metal (wt%) | Manganese (Mn) 3 wt% | Manganese (Mn) 3 wt% | Tin (Sn) 3 wt% | Manganese (Mn) 3 wt% | Manganese (Mn) 3 wt% | X |
| Second metal (wt%) | Zirconium (Zr) 0.1 wt% | Titanium (Ti) 0.1 wt% | Zirconium (Zr) 0.1 wt% | Zirconium (Zr) 0.6 wt% | X | Zirconium (Zr) 0.1 wt% |
| Light olefins (ethylene + propylene + butylenes + butadiene) (wt%) | 13 | 11 | 8 | 9 | 4.7 | 3.8 |
| Coke (wt%) | 15.5 | 16 | 15.6 | 15.2 | 24 | 19 |

As shown in Table 2, it could be confirmed that, in Examples 1 to 4, the light olefin conversion yield from the waste plastic pyrolysis oil and the coke reduction effect were more excellent compared to those in Comparative Examples 1 and 2.

Specifically, it could be confirmed that, in Example 1, as 3 wt% of manganese as a first metal and 0.1 wt% of Zirconium as a second metal were contained in the catalytic cracking catalyst, the light olefin conversion yield and the coke reduction effect were excellent.

It could be confirmed that, in Example 4, as the weight ratio of the first metal to the second metal was 6:1.2 (3 wt% of manganese and 0.6 wt% of Zirconium), the amount of light olefins produced was slightly lower than that in Example 1, but was excellent compared to those in Comparative Examples 1 and 2.

It could be confirmed that, in Example 2, as 3 wt% of manganese as a first metal and 0.1 wt% of titanium as a second metal were contained in the catalytic cracking catalyst, the light olefin conversion yield and the coke reduction effect were slightly deteriorated compared to those in Example 1, but were excellent compared to those in Comparative Examples 1 and 2.

It could be confirmed that, in Example 3, as 3 wt% of tin as a first metal and 0.1 wt% of Zirconium as a second metal were contained in the catalytic cracking catalyst, the light olefin conversion yield and the coke reduction effect were slightly deteriorated compared to those in Example 1, but were excellent compared to those in Comparative Examples 1 and 2.

On the other hand, it could be confirmed that, in Comparative Example 1, as only 3 wt% of the first metal (manganese) was contained and the second metal (Zirconium) was not contained in the catalytic cracking catalyst, the light olefin conversion yield was reduced, and the amount of coke produced was also increased.

It could be confirmed that, in Comparative Example 2, as the first metal was not contained and only 3 wt% of the second metal (Zirconium) was contained in the catalytic cracking catalyst, the light olefin conversion yield was the lowest.

As set forth above, light olefins may be obtained from waste plastic pyrolysis oil with a high yield by the method according to the present invention.

The olefin selectivity may be high and the production of coke may be minimized by the method according to the present invention.

**Claims**

1.   A method for converting waste plastic pyrolysis oil into light olefins, the method comprising:

(1) inputting waste plastic pyrolysis oil into a reactor;
(2) allowing the waste plastic pyrolysis oil to react in the reactor in the presence of a catalytic cracking catalyst containing a first metal and a second metal; and
(3) recovering light olefins by separating the catalytic cracking catalyst and oil from a product obtained in the step (2) .

2. The method of claim 1, wherein the first metal includes at least one metal selected from manganese, tin, and zinc.

3. The method of claim 1 or 2, wherein the second metal includes at least one metal selected from titanium, zirconium, hafnium, niobium, and vanadium.

4. The method of any one of claims 1 to 3, wherein the catalytic cracking catalyst further contains a zeolite, clay, and a binder.

5. The method of claim 4, wherein the zeolite includes ZSM-5, ZSM-11, Y-zeolite, ferrierite, mordenite, MCM-22, SUZ-4, or L-type zeolite.

6. The method of any one of claims 1 to 5, wherein a weight ratio of the first metal to the second metal in the catalytic cracking catalyst is 6:0.1 to 6:1, preferably 6:0.1 to 6:0.8, and more preferably 6:0.2 to 6:0.6.

7. The method of claim 4, wherein the catalytic cracking catalyst contains 20 to 70 wt%, preferably 30 to 50 wt% of the zeolite, 10 to 60 wt%, preferably 20 to 40 wt% of the clay, 10 to 50 wt%, preferably 15 to 35 wt% of the binder, 1 to 6 wt%, preferably 1 to 4 wt% of the first metal, and 0.1 to 1 wt%, preferably 0.1 to 0.6 wt% of the second metal.

8. The method of any one of claims 1 to 7, wherein the catalytic cracking catalyst has an average particle size of 50 to 2,000 pm, preferably 50 to 700 pm, more preferably 80 to 150 pm, as measured using a test method based on ASTM standard test method D4464-201.

9. The method of any one of claims 1 to 8, wherein the catalytic cracking catalyst has a total specific surface area of 50 to 150 $m^2/g$, preferably 70 to 100 $m^2/g$, and/or an apparent density of 0.5 to 1 $g/cm^3$, preferably 0.5 to 0.8 $g/cm^3$.

10. The method of any one of claims 1 to 9, wherein catalytic cracking catalyst satisfies the following Expression 1.

$$[Expression\ 1]$$

$$2 < (D90-D10)/D50 < 5$$

wherein D10 is a 10% cumulative diameter, D50 is a 50% cumulative diameter, and D90 is a 90% cumulative diameter, and wherein each of D10, D50 and D90 are as determined with laser diffraction according to DIN ISO 13320.

11. The method of any one of claims 1 to 10, wherein the waste plastic pyrolysis oil contains a vacuum gas oil (VGO) component having a boiling point of 340°C or higher at atmospheric pressure.

12. The method of any one of claims 1 to 11, wherein the reactor is a fluidized bed reactor.

13. The method of any one of claims 1 to 12, wherein the step (2) is performed at a temperature of 400 to 600°C and a reaction pressure of 50 to 200 kPa.

14. A device for converting waste plastic pyrolysis oil into light olefins, the device comprising:

a fluidized bed reactor configured to receive waste plastic pyrolysis oil and to perform a catalytic cracking reaction;
a cyclone configured to receive a product from the fluidized bed reactor and to separate the product into a catalyst and oil; and
a stabilizer configured to receive the oil from the cyclone and to separate the oil into a gas component and a liquid component.

15. The device of claim 14, wherein the device is configured for carrying out a method of any one of claims 1 to 13.